# EUROPEAN PATENT APPLICATION

(11) **EP 4 309 682 A1**
(43) Date of publication of application: **24.01.2024**
(21) Application number: 21931643.7
(22) Date of filing: 22.04.2021
(51) Int. Cl.: A61L 9/01

(54) **INDOOR AIR CLEANER**

(30) Priority: 19.03.2021 JP 2021045428; 19.04.2021 JP 2021070304
(71) Applicant: Shinkokiki Co., Ltd., Nagoya-shi Aichi 452-0822 (JP)
(72) Inventor: FUKIZAWA Takeo, Nagoya-shi, Aichi 452-0822 (JP)
(74) Representative: Mewburn Ellis LLP
(86) International application number: PCT/JP2021/016342
(87) International publication number: WO 2022/195898

(57) **Abstract**

In an indoor air cleaner according to the present invention, shavings 10 generated when a copper material is cut with a lathe or milling machine are stored inside a breathable net 11. The copper material may be pure copper or various copper alloys. The net 11 may be formed into a spherical shape and hung, or the net may be formed into a container and placed on a desk or the like. When airborne bacteria and viruses in a room come into contact with the surfaces of the shavings 10, the antibacterial and antiviral effects of copper can reduce the activity of the bacteria and viruses.

## Description

### TECHNICAL FIELD

The present invention relates to an indoor air cleaner using antibacterial and antiviral effects of copper.

### BACKGROUND ART

It is conventionally widely known that copper has antibacterial and antiviral effects. The mechanism is thought to be that copper ions make holes in viral envelope membranes or that free radicals produced by copper ions degrade genetic material.

According to a research team at the National Institutes of Health in the United States, the new coronavirus survives for 72 hours on plastic and stainless steel surfaces and for 24 hours on cardboard surfaces, whereas it survives for 4 hours on copper surfaces.

According to Japan Copper Development Association, it has been proven that copper has excellent antibacterial and antiviral effects on fungi such as Pseudomonas aeruginosa, Escherichia coli, and Legionella, Cryptosporidium, and influenza virus and norovirus.

Antibacterial materials using such antibacterial and antiviral effects of copper have conventionally been proposed. For example, Patent Literature 1 describes dispersion of very fine copper alloy powder with a particle diameter of 1 nm to 1 um on a surface of an article. Patent Literature 2 describes an antibacterial fabric made of composite fibers including copper alloy yarn. Patent Literature 3 describes an antibacterial cement containing copper powder mixed in Portland cement.

Unfortunately, the antibacterial materials described in these related patent literatures involve high production costs and cannot be provided with low prices, because copper is processed into minute powder or minute fibers in order to increase the surface area. Furthermore, in the material containing copper powder kneaded into resin or cement, only the copper powder exposed on the surface contributes to the antibacterial and antiviral effects, while the copper powder buried in the resin or cement is wasted.

There are commercially available air cleaners that suck the indoor air with a fan and feed the sucked air into a UV sterilization chamber to reduce the activity of bacteria and viruses. However, these air cleaners require electricity for operation, and strong ultraviolet rays may have adverse effects on humans and pets, and thus they must be handled with care.

### CITATION LIST

### Patent Literature

Patent Literature 1: Japanese Unexamined Patent Application Publication No. 2019-065375
Patent Literature 2: Japanese Unexamined Patent Application Publication No. 2019-143272
Patent Literature 3: Japanese Unexamined Patent Application Publication No. 2006-249041

### SUMMARY OF INVENTION

### Technical Problems

An object of the present invention is therefore to solve the above conventional problems and provide an indoor air cleaner that can be manufactured inexpensively and is highly safe with no risk of adverse effects on the human body or pets.

### Solutions to Problems

The inventor of the present invention is engaged in processing of copper products and has noted a large amount of shavings generated when cutting copper materials with lathe or milling machines. These shavings have conventionally been disposed of as waste. These shavings are thin and spiral or thin strips and have a large surface area. The inventor has conceived that these shavings can be used to provide an indoor air cleaner using the antibacterial and antiviral effects of copper inexpensively.

An indoor air cleaner according to the present invention has been completed based on the above finding and is characterized in that shavings generated when a copper material is cut are stored inside a breathable net.

According to a preferred embodiment, the shavings are spiral shavings generated when a copper material is lathed, and are stored in a compressed and shaped state inside the net. According to another preferred embodiment, the shavings are shavings generated when a copper material is milled.

Furthermore, it is preferable that the net is a stainless steel, and the net may be spherical and capable of hanging indoors with a hanging string. The net may be shaped like a container and formed as a decorative object to be placed indoors with an artificial flower at a top of the container.

### Advantageous Effects of Invention

The indoor air cleaner according to the present invention can be manufactured extremely inexpensively because it utilizes shavings generated when a copper material is cut with a lathe or milling machine, which have conventionally been disposed of as waste. The shavings, which are lightweight, yet have large surface areas, are stored inside the breathable net to allow the air to circulate while avoiding scattering of the shavings. This configuration allows airborne bacteria and viruses to come into contact with the copper surfaces and reduces their activity.

Copper is a metal safe for the human body as used for pots and dishes from ancient times, and thus even if an infant touches or licks it, there will be no adverse effect on the human body. Furthermore, the indoor air cleaner according to the present invention is advantageous in that it only uses the antibacterial and antiviral effects of copper ions and thus the effect lasts semipermanently. In addition, the indoor air cleaner according to the present invention needs only to be placed indoors and requires no electricity.

### BRIEF DESCRIPTION OF DRAWINGS

FIG. 1 is an external view of shavings generated in lathing.
FIG. 2 is an external view of a compressed body of shavings.
FIG. 3 is an external perspective view of an indoor air cleaner of a first embodiment.
FIG. 4 is an external perspective view of an indoor air cleaner of a second embodiment.
FIG. 5 is an external perspective view of the indoor air cleaner of the second embodiment.
FIG. 6 is a plan view illustrating showing the indoor air cleaner with the crossbar moved.
FIG. 7 is an external view illustrating a watering state.

### DESCRIPTION OF EMBODIMENT

A first embodiment of the present invention will be described below.

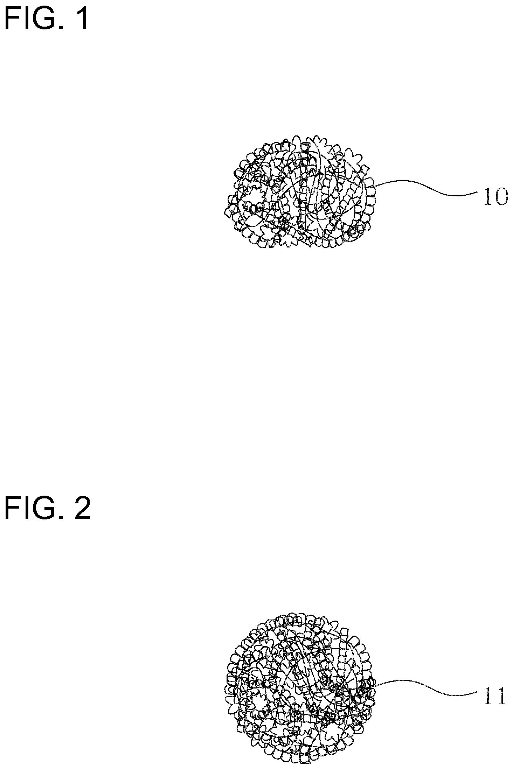

As illustrated in FIG. 1, shavings 10 generated when a copper material is cut with a lathe machine are in the form of fine spirals with a width of approximately 1 to 10 mm and a thickness of approximately a few um to a few tens of µm, depending on the cutting state and the type of copper material. Such shavings 10 are lightweight, yet have a large surface area, and have an intricate interior space to allow airborne bacteria and viruses to come into contact with copper surfaces thereby reducing their activity.

The copper material used in the present invention may be pure copper or a copper alloy. Examples of the copper alloy include various copper alloys, such as alumina dispersion strengthened copper, silver-containing copper, beryllium-containing copper, chromium zirconium copper, and cobalt-containing copper. Since these copper alloys are widely used as industrial materials, a large amount of shavings 10 is generated therefrom and readily and inexpensively available. The antibacterial and antiviral effects are derived from copper ions, and alloy components except silver are not expected to have antibacterial and antiviral effects. However, these alloy components do not inhibit the antibacterial and antiviral effects of copper.

Since the spiral shavings 10 generated when a copper material is lathed are spiral, elastic, and difficult to assemble, it is preferable that the shavings 10 are processed into a compressed body 11 as illustrated in FIG. 2. This processing can be easily performed by pressing the shavings 10 placed in a mold. The compressed body 11 is spherical in FIG. 2 but may have any shape such as a rectangular parallelepiped or a plate-like shape. The shavings 10, compressed in this way, are entangled with each other and plastically deformed to keep a certain shape. If the pressing pressure is too high, the shavings 10 come into close contact with each other and are reduced in surface area and impaired in air permeability, and thus it is necessary to compress the shavings 10 to such an extent as not to be completely crushed.

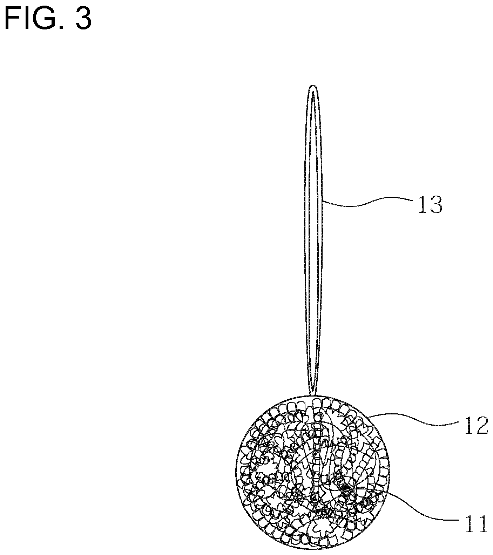

As illustrated in FIG. 3, in the first embodiment, the compressed body 11 is stored in a net 12. The material of the net 12 is not limited as long as it is breathable and strong. In this embodiment, a stainless steel net with excellent durability is used. The mesh size of the net 12 is set such that there is no danger of the edges of the shavings 10 protruding to the outside.

In the first embodiment illustrated in FIG. 3, a hanging string 13 is attached to the net 12, so that it can be hung indoors. When hung near a window, near a vent, or in any other airflow path, the indoor air cleaner can catch airborne bacteria and viruses on the copper surfaces of the shavings 10 and reduce their activity.

This indoor air cleaner uses shavings that have conventionally been disposed of as waste, therefore can be manufactured extremely inexpensively, and provides excellent antibacterial and antiviral effects and deodorizing effects. The indoor air cleaner is also advantageous in that it is safe for the human body and can keep the antibacterial and antiviral effects semipermanently.

A second embodiment of the present invention will now be described.

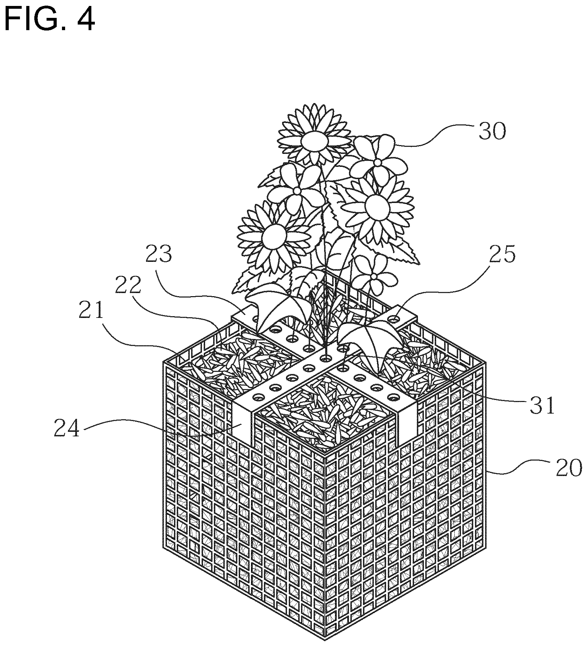

FIG. 4 is an external perspective view of the second embodiment, illustrating a container 20 formed of a strong and breathable net 21. The material of this net 21 is preferably, but not limited to, a metal in terms of strength and rigidity, and in the present embodiment, a stainless steel net that is unlikely to rust. In the embodiment, a flat plate-shaped stainless steel net is used to form sides and a bottom to make a cube-shaped container 20 with each side of 10 to 15 cm. The top of the container 20 is open, and the bottom is not necessarily a net. The inside of such a container 20 is filled with copper shavings 22.

The shavings 22 illustrated in FIG. 4 are shavings generated when a copper material is cut with a milling machine, and mostly shaped like thin strips although the shape varies depending on the cutting conditions and the type of copper material. It is preferable that sieving is performed, if necessary, so that the container 20 is filled with those of a substantially constant size. The shavings 22 can be put in the container 20 as they are or can be compressed into a certain shape as in the first embodiment to be fitted into the container 20.

Since the shavings 22 filled in the container 20 have numerous voids between each other, the contact area between the copper surfaces and the air is extremely large. Moreover, the voids formed inside the shavings 22 have intricate shapes, so that airborne bacteria and viruses can be certainly caught on the copper surfaces of the shavings 22, in the same manner as a mask. Thus, the effect of reducing the activity of airborne bacteria and viruses is increased. Moreover, unlike copper powder or copper fibers, such shavings 22 do not scatter in the air and are not accidentally inhaled. Copper is a safe metal used in pots, pans, and the like, and thus even if an infant accidentally touches or licks it, there will be no adverse effects on the human body.

Although the effect of purifying indoor air is achieved simply by filling the interior of the container 20 with the copper shavings 22, in the present invention, a decorative element 30 is disposed at the top of this container 20 to make an indoor decorative object. In this embodiment, a crossbar 23 is put across two opposing sides of the container 20 in a quadrangular shape. The crossbar 23 is a metal plate of stainless steel or the like with ends 24 bent downward and also functions to prevent deformation of the container 20.

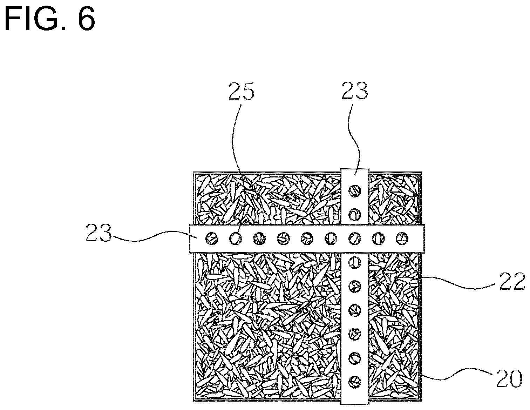

As illustrated in the figure, the crossbar 23 has a number of mounting holes 25 for inserting the decorative element 30. The crossbar 23 may be placed at a central position of the container 20 as illustrated in FIG. 4, or may be slid into a position as desired as illustrated in FIG. 6.

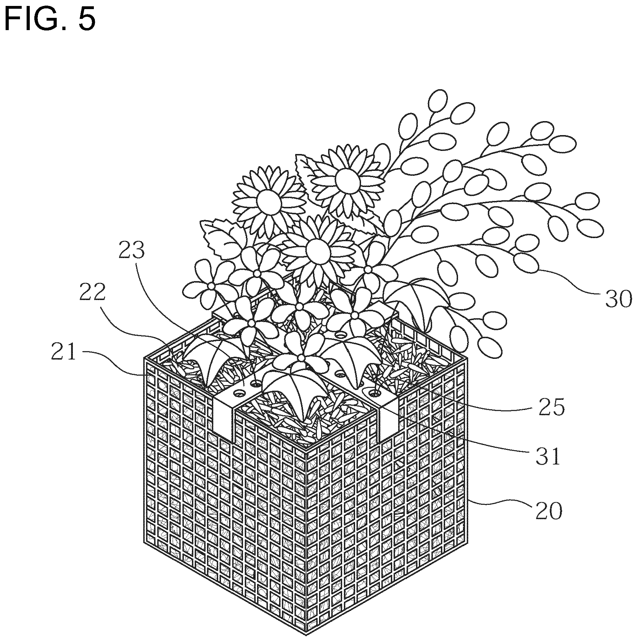

In FIG. 4, an artificial flower is used as the decorative element 30, and a central axis 31 of the artificial flower is inserted into the mounting hole 25 of the crossbar 23. However, as illustrated in FIG. 5, the central axis 31 of the artificial flower can be inserted into a gap in the shavings 22 through a section without the crossbar 23. Disposing artificial flowers at the top of the container 20 in this way can make an appearance that resembles flowers planted in a flowerpot. However, the decorative element 30 is not limited to artificial flowers and may be changed to dolls, flags of all nations, or the like, if necessary. The shape of the container 20 may be square as in the above embodiment or may be cylindrical.

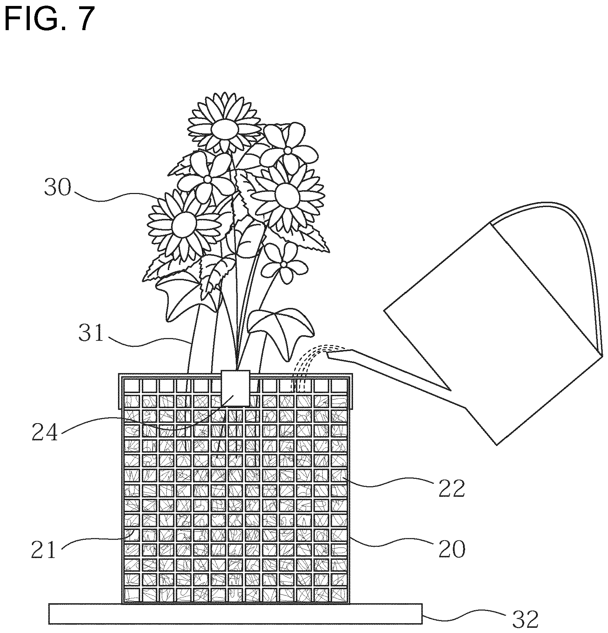

The indoor air cleaner according to the second embodiment can be simply placed on a desk or the like to purify indoor air. A small amount of water may be poured as in a flowerpot, as illustrated in FIG. 7. Although the copper shavings 22 are not water-absorbent, some of the water adheres to the surfaces of the shavings 22 and the remainder flows down through the gaps in the shavings 22. For this, it is desirable to place a water-absorbing pad 32 or a saucer at the bottom of the container 20.

Since copper ions exhibit the antibacterial effect even on microorganisms in water, the water coming into contact with the shavings 22 is kept clean and free of bacteria and mold. The poured water gradually evaporates to humidify the room. The shavings 22 are expected to be more effective in catching bacteria and viruses in the air when the shavings 22 have water adhering to the surfaces than when they are dry. For this reason, it is preferable to pour a small amount of water every morning in the same manner as when water is poured into a flowerpot.

As explained above, the present invention can provide an indoor air cleaner that can be manufactured inexpensively and is excellent in safety and persistent antibacterial and antiviral effects.

### REFERENCE SIGNS LIST

- 10: shavings
- 11: compressed body
- 12: net
- 13: hanging string
- 20: container
- 21: net
- 22: shavings
- 23: crossbar
- 24: end
- 25: mounting hole
- 30: decorative element
- 31: central axis of artificial flower
- 32: water-absorbing pad

## Claims

1. An indoor air cleaner comprising shavings generated when a copper material is cut stored inside a breathable net.

2. The indoor air cleaner according to claim 1, wherein the shavings are spiral shavings generated when a copper material is lathed, and are stored in a compressed and shaped state inside the net.

3. The indoor air cleaner according to claim 1, wherein the shavings are shavings generated when a copper material is milled.

4. The indoor air cleaner according to any one of claims 1 to 3, wherein the net is a stainless steel net.

5. The indoor air cleaner according to any one of claims 1 to 4, wherein the net is spherical and capable of hanging indoors with a hanging string.

6. The indoor air cleaner according to any one of claims 1 to 4, wherein the net is shaped like a container and formed as a decorative object to be placed indoors with an artificial flower at a top of the container.
